# EUROPEAN PATENT SPECIFICATION

(11) **EP 2 224 998 B1**
(45) Date of publication and mention of the grant of the patent: **26.10.2011**
(21) Application number: 07852148.1
(22) Date of filing: 21.12.2007
(51) Int. Cl.: A61N 1/05

(54) **IMPLANTABLE MEDICAL LEAD**
IMPLANTIERBARE MEDIZINISCHE LEITUNG
SONDE MÉDICALE IMPLANTABLE

(43) Date of publication of application: 08.09.2010
(73) Proprietor: St. Jude Medical AB, 175 84 Järfälla (SE)
(72) Inventor: HILL, Rolf, S-175 55 Järfälla (SE); STEGFELDT, Olof, S-138 57 Älta (SE)
(86) International application number: PCT/SE2007/001154
(87) International publication number: WO 2009/082283

(56) References cited:
- EP-A1- 1 774 986
- WO-A1-2007/064263
- WO-A1-2007/123443
- US-A1- 2005 070 984
- US-B1- 7 092 766

## Description

### TECHNICAL FIELD

The present invention generally relates to implantable medical leads, and in particular to active fixation implantable medical leads.

### BACKGROUND

Body implantable electrical leads form the electrical connection between an implantable medical device (IMD), such as cardiac pacemaker, cardiac defibrillator or cardioverter, and body tissue, such as the heart, which is to be electrically stimulated. As is well known, the leads connecting the IMD with the tissue may be used for pacing/defibrillation and for sensing electrical signals produced by the tissue.

The implantable leads of today and in particular cardiogenic implantable leads can generally be divided into two classes depending on the tissue anchoring arrangement of the leads. Firstly, so-called passive fixation leads comprise radially protruding elements in the distal lead ends. These elements can become embedded in the trabecular network inside the heart and thereby provide an anchoring of the lead to the heart tissue. Examples of such protruding elements include collars, tines and fines. Passive leads are generally characterized by low chronic capture threshold and high impedance.

The other class of leads includes so-called active fixation leads. Such a lead typically comprises, in its distal end, a helical fixation element that can be screwed into the endocardium and myocardium to provide the necessary lead-to-tissue anchoring. Generally, active leads have superior ability to fixate without the need for any trabecular network.

The helical fixation element is generally extendable and retractable relative the distal lead end by providing a fixed post projecting radially inwardly from the lead header. By then providing a rotating motion at a connector pin in the proximal end of the lead, the rotational movement is transferred via an inner conductor and helix shaft up to the helical fixation element. The rotation of the fixation element forces, due to the post, the element to advance or retract within the lead header. Such a lead solution is disclosed by U.S. Patent No. 7,092,766.

U.S. Patent No. 6,819,959 discloses another guiding solution for allowing a helical fixation element to advance and retract within the lead header. In this case, the post has been moved from the inner surface of the header to the helix shaft. This shaft therefore has a radially outwardly projecting post. A spiral track element is provided in the lead header and adapted to engage with the shaft post during an applied rotation to thereby transform the rotational movement into a longitudinal movement of the helical fixation element.

WO-A-2007/123443 is the closest prior art to the present invention and discloses a post that is attached to the tubular header assembly by welding and thus fails to disclose a post having a post head mechanically supported by the lateral surface of the tubular header assembly and a protruding element connected to the post head and extending through the radial aperture.

The present invention solves the problem of simplifying the manufacture of an implantable medical lead.

### SUMMARY

The above disclosed prior art solutions for transforming a rotational movement into a longitudinal movement using a header post or a shaft post imposes a drawback during lead manufacturing. The prior art lead headers consist of a multitude of lead elements that have to be interconnected in, often rather complex and time-consuming, assembling operations. It is therefore a need for an active fixation lead design that simplifies manufacture and assembly.

The present invention overcomes these and other drawbacks of the prior art arrangements.

It is a general object of the present invention to provide an implantable medical lead.

It is another object of the invention to provide a simplified manufacturing process for an implantable medical lead.

These and other objects are met by the invention as defined by the accompanying patent claims.

Briefly, the present invention involves an implantable medical lead adapted for connection to an implantable medical device. The lead of the present invention is a so-called active fixation lead implying that the lead comprises a helical active fixation element for attaching the lead to a tissue to be stimulated and/or sensed by the implantable medical device.

The lead of the invention comprises a tubular header assembly defining an inner header channel running from a first short end to a second opposite short end of the header assembly. A radial aperture is provided or formed in the assembly for providing a radial access into the inner channel. An inner lead assembly is coaxially housed within the inner header channel. This inner lead assembly comprises the helical active fixation element connected to an inner conductor, preferably through a helical shaft. The inner conductor runs along the length of the lead body and ends at the opposite end of the helical shaft as compared to the fixation element.

Once the inner assembly has been fully introduced into the channel, a post is inserted into the radial aperture, projecting radially inwardly into the inner header channel to become interposed between adjacent turns of the helical fixation element. The post is used for transforming a rotational movement of the inner lead assembly to a longitudinal movement of the helical fixation element allowing the helical element to advance out from or retract into header assembly.

The radial aperture can be formed by penetrating the wall of the header assembly by the post through a pushing or screwing action.

By having an insertable post instead of a fixed post anchored to the inside surface of the header, the inner lead assembly can be pre-manufactured before lead assembly and be handled as a single unit that is inserted into the header assembly. The post then, in addition to providing the rotational-to-longitudinal movement transformation, prevents the inner lead assembly from exiting the header assembly.

A post locking element may be circumferentially disposed around at least the portion of the header assembly comprising the radial aperture to prevent the post from leaving the aperture.

The invention offers the following advantages:
- Fewer lead components;
- Simplified lead assembly process; and
- Improve yield due to lower component contamination.

Other advantages offered by the present invention will be appreciated upon reading of the below description of the embodiments of the invention.

### SHORT DESCRIPTION OF THE DRAWINGS

The invention together with further objects and advantages thereof, may best be understood by making reference to the following description taken together with the accompanying drawings, in which:
Fig. 1 is a side view of an implantable lead according to an embodiment of the present invention;
Fig. 2 is a schematic overview of a subject having an implantable medical device connected to an implantable lead according to an embodiment of the present invention;
Fig. 3 is a view of header assembly according to the present invention equipped with a distal marker ring;
Fig. 4 is a view of an inner lead assembly with helical active fixation element, helix shaft and inner conductor;
Fig. 5 is a view of the inner lead assembly of Fig. 4 coaxially inserted into a header assembly of Fig. 3 further equipped with a header cap;
Fig. 6A is a view of the lead sub-assembly of Fig. 5 with a post inserted in a radial aperture and an inner insulating tubing provided around the inner conductor;
Fig. 6B is a cross-sectional view of the lead sub-assembly of Fig. 6A;
Fig. 7 is a view of a crimp sleeve connected to an outer conductor;
Fig. 8A is a view of the crimp sleeve and outer conductor connected to a ring electrode;
Fig. 8B is a cross-sectional view of the ring electrode assembly of Fig. 8A;
Fig. 9 is a view of the lead header in which the ring electrode assembly of Fig. 8A is being partly slid over the lead sub-assembly of Fig. 6A;
Fig. 10 is a view of the lead header in which the ring electrode assembly of Fig. 8A is fully slid on the lead sub-assembly of Fig. 6A to lock the post;
Fig. 11A is a view of the finally assembled lead header with an insulating and protecting sheath;
Fig. 11B is a cross-sectional view of the lead header of Fig. 11A;
Fig. 12 is a cross-sectional view of a lead header according to another embodiment of the present invention;
Fig. 13A is a side-view of a post embodiment according to the present invention;
Fig. 13B is an upper view of the post of Fig. 13A according to an embodiment of the present invention;
Fig. 13C is an upper view of the post of Fig. 13A according to another embodiment of the present invention;
Fig. 14 is a marker ring equipped with a post embodiment according to the present invention;
Fig. 15 is a flow diagram illustrating a method of manufacturing an implantable medical lead according to the present invention;
Fig. 16 is a flow diagram illustrating an additional step of the manufacturing method of Fig. 15; and
Fig. 17 is a flow diagram illustrating an additional step of the manufacturing method of Fig. 15.

### DETAILED DESCRIPTION

Throughout the drawings, the same reference characters will be used for corresponding or similar elements.

The present invention relates to implantable active fixation leads or catheters. The leads of the invention are connectable to different implantable medical devices (IMDs), such as pacemakers, cardioverters, defibrillators and other implantable electrical medical devices.

As is well known in the art, "proximal" relates, when applied to medical leads and their including components, to the lead/component portion facing the implantable medical device. Correspondingly, "distal" refers to the lead/component portion facing the tissue, in which the lead is (to be) anchored.

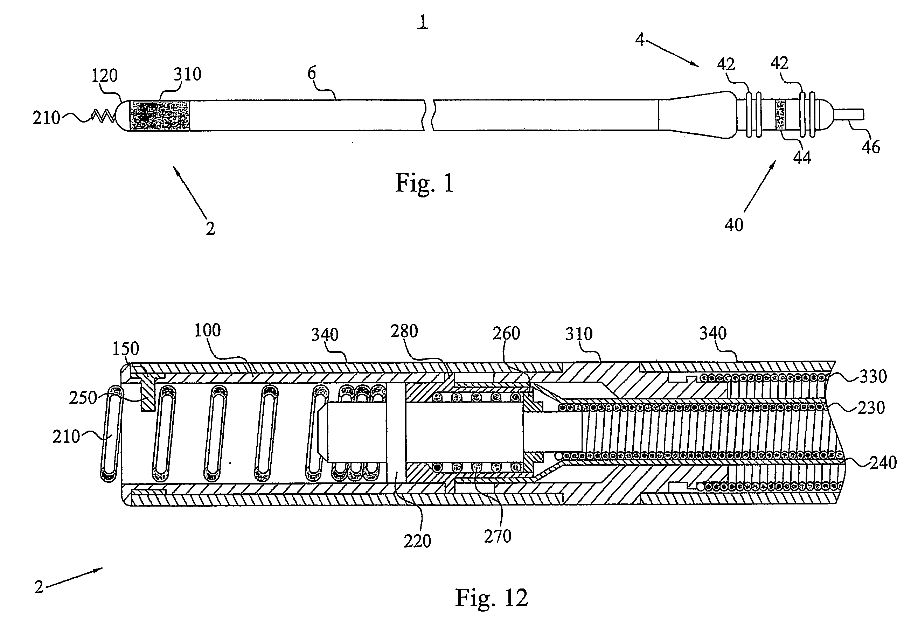

Fig. 1 is a schematic illustration of an implantable lead 1 according to an embodiment of the present invention. The lead 1 comprises a lead body 6 extending along a central, longitudinal axis. The lead 1 has a proximal end 4 carrying a connector assembly 40 for electrically connecting the lead body 6 to an IMD. The lead 1 also has a distal end 2 comprising a header with a header cap 120, a ring electrode 310 and a helical fixation element 210. The fixation element 210 is in the form of a helical, screw-in fixation element 210 adapted to be extended so as to project from the distal end of the lead 1. The helical screw-in fixation element 210 is preferably active electrically so as to function as an electrode when implanted to stimulate selected tissue, such as cardiac tissue, and/or sense electrical activity of the tissue. Consistent with teachings well known in the art, one or more portions of such a helical electrode 210 may be electrically insulated along its length. The helical electrode 210 not only has a stimulating and/or sensing function but also serves to anchor or stabilize the distal lead portion 2 relative to the tissue.

The distal lead portion 2 also preferably comprises a ring electrode 310, also denoted indifferent electrode in the art. This electrode 310 is provided for electrically stimulating adjacent tissue and/or for sensing electrical activity of tissue.

The connector assembly 40 at the proximal lead end 4 is adapted to electrically and mechanically couple the lead body 6 to the IMD. The assembly 40 comprises terminal contacts in the form of a tubular, rotatable pin terminal contact 46, often denoted connector pin, and a ring terminal contact 44, generally referred to as connector ring 44. These two contacts 44, 46 are positioned to engage corresponding electrical terminals within a receptacle in the IMD. In order to prevent ingress of body fluid into the IMD receptacle, the connector assembly 40 may be provided with spaced-apart sets of seals 42, well known in the art.

The lead 1 described above and present in Fig. 1 is a so-called far-field signal reduction (FSR) lead having a very short distance between the lead tip and the ring electrode 310. The teachings of the present invention is though, as is disclosed further herein, not limited to this particular design of implantable medical leads but can also be used in leads having larger tip-to-ring electrode distance and indeed to implantable leads lacking a ring electrode. Such leads may also or instead comprise one additional electrode, a so-called tip electrode positioned at the most distal end of the lead, basically at the position of the header cap in Fig. 1. The tip electrode is typically electrically interconnected with the helix electrode. The invention can also be used in implantable medical leads comprising more than one ring electrode.

The present invention is furthermore not limited to electrically conducting helical screw-in fixation elements. In clear contrast, the teachings of the invention can be applied to helical screw-in fixation elements that are not employed as stimulating/ sensing electrodes, i.e. being made of nonconducting material and/or having no electrical contact with electrical terminals in the IMD.

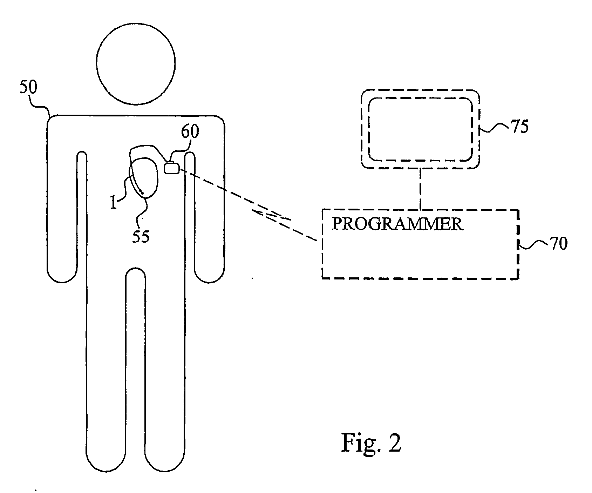

Fig. 2 is a schematic overview of a subject 50 equipped with an IMD 60 connected to the subject's heart 55. The IMD 60 is illustrated as a device that monitors and/or provides therapy to the heart 55 of the patient 50, such as a pacemaker, defibrillator or cardioverter. However, the present invention is not limited to cardiac-associated IMDs but may also be practiced with other implantable medical devices, such as drug pumps, neurological stimulators, physical signal recorders, oxygen sensors, or the like, as long as the IMD 60 is equipped with or is connected to at least one medical lead 1 according to the present invention. For this purpose the IMD 60 comprises a lead connecting arrangement that mechanically and electrically, through electrode terminals in the connecting arrangement, connects the lead 1 and its electrodes with the IMD 60.

The IMD 60 can wirelessly communicate with an external device 70, non-limitedly illustrated as a programmer 70 in the figure. The external device 70 could alternatively be a physician's workstation, a home monitoring device, a base station or actually any data processing unit having capability of receiving data collected by the IMD 60 and preferably sending instructions and commands to the IMD 60. The external device 70 is preferably connected to a display screen 75 allowing display of the collected diagnostic parameters and data

It is anticipated by the present invention that a lead or catheter of the present invention does not necessarily have to be used in a human subject but can instead be implantable in other animal subjects, in particular mammalian subjects.

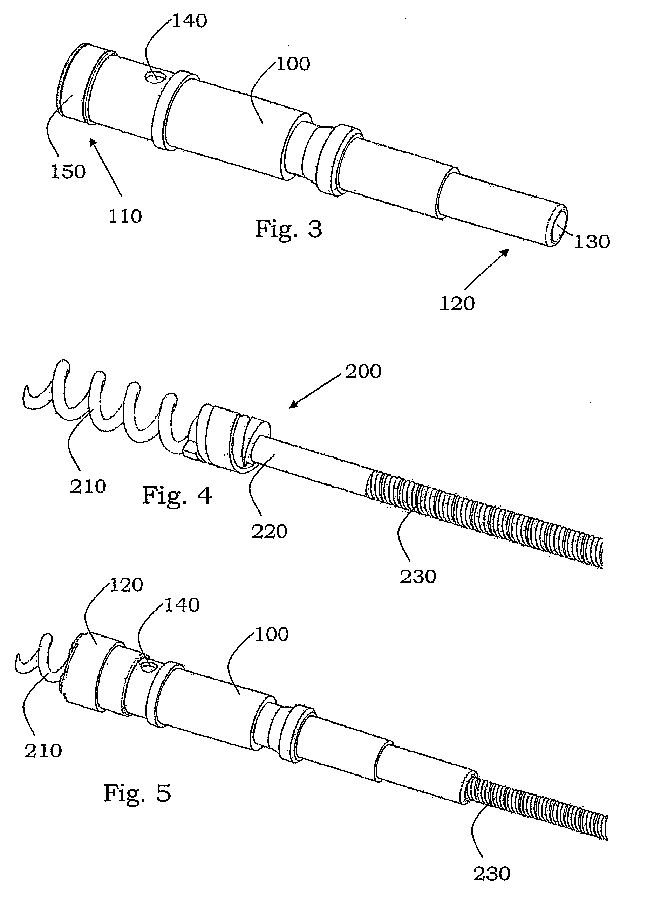

Figs. 3 to 11B illustrate a manufacturing and assembly process with the different lead components present in the distal end of an FSR lead.

Fig. 3 is a schematic view of a tubular header assembly 100 according to the present invention. The header assembly defines an inner header channel or bore 130 running longitudinally from a first or distal short end 110 of the assembly 100 to an opposite second or proximal short end 120. The header assembly 100 is preferably in the form of a hollow cylinder or tube made of an electrically insulating material, such as polyetheretherketone (PEEK), silicone, polyurethane or the like.

In clear contrast to the prior art header assemblies, such as illustrated in US 7,092,766, the header assembly 100 does not comprise any fixed post projecting radially inwardly from an inner surface of the assembly 100 and towards the central longitudinal axis of the channel 130. The header assembly 100 instead comprises a radial aperture 140 forming a whole in the lateral surface of the assembly 100 to thereby provide access to the inner channel 130 from the outside. The radial aperture 140 is preferably provided somewhere in the most distal half of the header length. Basically, the radial aperture 140 should be provided over a position that the helical active fixation element occupies when being inserted into the inner channel 130.

The header assembly 100 does not necessarily have to be pre-manufactured with the radial aperture 140. In clear contrast, the aperture 140 can be formed by a post penetrating the header wall, as is described further below.

An optional marker ring 150 made of a radiopaque material, such as platinum, iridium, gold, tantalum or an alloy thereof, can be provided in connection with the distal end 110 of the header assembly 100. The marker ring 150 can be employed by a physician for facilitating guiding the lead into correct position during implantation by being visual in radiological imaging and X-ray images. Furthermore, the marker ring 150 is used for visualizing the position of the helix 210 relative the most distal lead end. Thus, the marker ring 150 is employed, typically together with a radiopaque portion of a helix shaft, for visualizing the lateral screwing motion of the helix 210 during tissue anchoring.

The header assembly 100 preferably comprises a dedicated waist or groove in which the marker ring 150 is inserted to thereby being prevented from any movement along the central axis of the assembly 100 by the radial protrusion defining the waist or groove.

The proximal end 120 of the header assembly 100 preferably has a somewhat smaller outer diameter as compared to the distal end 110. As a consequence, an inner insulating tubing can be slid onto the proximal header portion, which is illustrated further herein.

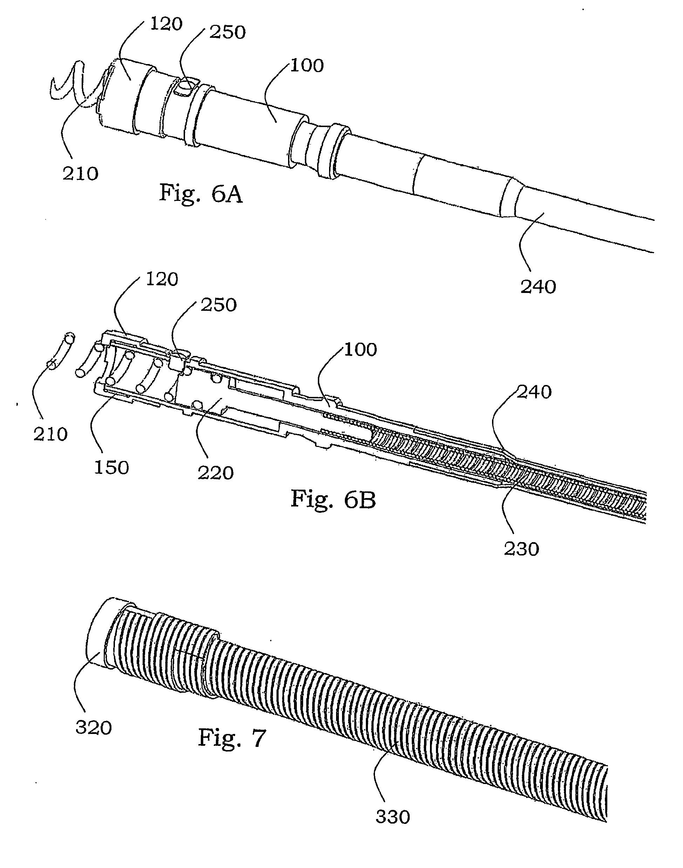

The diameter of the inner channel 130 preferably differs between the distal header portion 110 and the proximal header portion 120 as is better seen in Fig. 6B. The distal portion of the channel 130 is adapted for housing the helical fixation element and a distal portion of a helical shaft, whereas the proximal channel portion contains the proximal shaft portion and an inner conductor. As the helical fixation element has an outer diameter that exceeds the outer diameter of the inner conductor, the channel diameter at the distal header portion 110 is larger than the diameter at the proximal header portion 120.

The transition from larger channel diameter to smaller channel diameter defines a stop for the helical fixation element and the shaft. This stop prevents the fixation element from being moved further into the lead.

Fig. 4 is an illustration of an inner lead assembly 200 according to the present invention. This inner lead assembly 200 comprises the helical active fixation element 210, preferably made of an electrically conducting material, such as platinum, iridium, gold, tantalum, titanium or an alloy such as platinum/iridium, MP35N^{®} or stainless steel. The distal helix end is adapted for insertion into a tissue, such as myocardium to thereby anchor the lead thereto. The other opposite helix end is connected to a first or distal end of a helix shaft 220 providing a mechanical and preferably electrical bridge between the fixation element 210 and an inner conductor 230. The shaft material can be selected from the above-presented materials for the fixation element and is preferably made of the same material as the helix 210. The helix-shaft connection can be realized through different known connection techniques, such as welding. The distal shaft portion can preferably be equipped with threads allowing a portion of the helical fixation element 210 to be screwed onto the shaft 220 as is illustrated in the figure.

The inner conductor 230 is preferably in the form of an inner conductor coil 230 running along the length of the lead to thereby provide electrical connection between the helical fixation element and a terminal electrode at the proximal lead end. The conductor 230 is also mechanically connected to the shaft, such as by welding, to transfer a rotational movement from the proximal lead end up to the helical fixation element 210. Suitable materials for the inner conductor can be found among the list given above for the fixation element 210.

In another embodiment of the inner lead assembly 200, the helical shaft is omitted. This implies that the helical fixation element 210 is mechanically and preferably electrically connected directly to inner conductor 230. The most distal portion of the conductor 230 is preferably threaded over the most proximal helical fixation element portion. The two lead elements 210, 230 can then be welded together.

As the helical fixation element 210, optional shaft 220 and conductor 230 are mechanically connected, the inner lead assembly 200 can be handled as a single unit during the lead assembly. This inner lead assembly 200 can therefore be pre-assembled into a single unit before the lead is to be put together.

The reason for allowing this pre-manufacturing of the inner lead assembly 200 is that the header assembly does not have any fixed, inwardly protruding post as in the art. As a consequence, the whole inner lead assembly can be freely slid into the header assembly 100, see Fig. 5, and the header channel from the distal header end. Remember that the channel diameter at the proximal header end is smaller than the outer diameter of the fixation element and thereby prevents the inner lead assembly 200 from being inserted into the channel 130 from this side.

If the header assembly 100 would have had a fixed post as in the prior art, it would not have been possible to have a single inner lead assembly 200 and introduce it into the channel 130 as the post prevents this insertion. In clear contrast, the header assembly would then have to be divided into a separate header part and a separate coupling part that have to be connected during the assembly. The present invention therefore allows usage of fewer lead elements and a by far simpler assembly process.

Fig. 5 illustrates the inner lead assembly coaxially housed within the inner header channel of the header assembly 100. At this position, the radial aperture 140, if pre-formed in the header 100, is positioned over the helical fixation element 210 and the inner conductor 230 projects out from the inner channel.

The figure also illustrates an optional header cap 120 attached to the most distal header end to thereby cover the marker ring. The header cap 120 is preferably made of an insulating material, such as silicone, PEEK or polyurethane.

A post 250 according to the present invention is then inserted into the radial aperture 140 in Figs. 6A and 6B. At this inserted position, the post 250 will project radially inwardly into the inner header channel. Furthermore, the post 250 is preferably interposed between adjacent turns of the helical fixation element 210.

As is illustrated in Fig. 6A, the post 250 preferably comprises a post head mechanically supported by the lateral surface of the header assembly 100. At this position, a protruding element connected to the post head extends through the radial aperture 140 and projects radially inwardly into the channel.

Optionally, the header assembly 100 comprises an indentation in the lateral surface in connection with the radial aperture 140. The shape of the indentation then preferably matches the shape of the post head.

If the header 100 does not have a pre-manufactured aperture, the post 250 itself can be used as a penetrating tool for forming the aperture 140 as the post 250 is pushed or screwed through the header wall.

For sealing purposes a silicone adhesive, or the like, can be applied over the post head to form a sealing layer thereon, preventing contamination of the helix during the assembly process. Alternatively, or in addition a silicone ring or silicone adhesive, or the like, can be applied between the header assembly 100 and the post 250 to form a sealing once the post 250 is pushed into the radial aperture. This further minimizes, in the case of FSR leads having a ring electrode coaxial with and positioned radially around the helix electrode, helix contamination during the assembly process.

The post 250 is, for the purpose of FSR leads, preferably made of an insulating material, such as PEEK, silicone or polyurethane.

The figures also illustrates an inner insulating tubing 240 provided coaxially around the inner conductor 230 and preferably being thread onto the proximal header portion. Different insulating materials, such as silicone, polyurethane and PTFE, can be used as insulating tubing material.

Fig. 7 is an illustration of an outer conductor (coil) 330 connected to an optional coil adapter or crimp sleeve 320. The conductor coil 330 is made of an electrically conducting material, such as platinum, iridium, gold, tantalum, titanium or an alloy such as platinum/iridium or MP35N^{®}. The crimp sleeve 320 is also made of a conducting material, preferably the same material as the outer coil 330. These two conducting elements 320, 330 may, but does not necessarily have to, be connected to each other through welding.

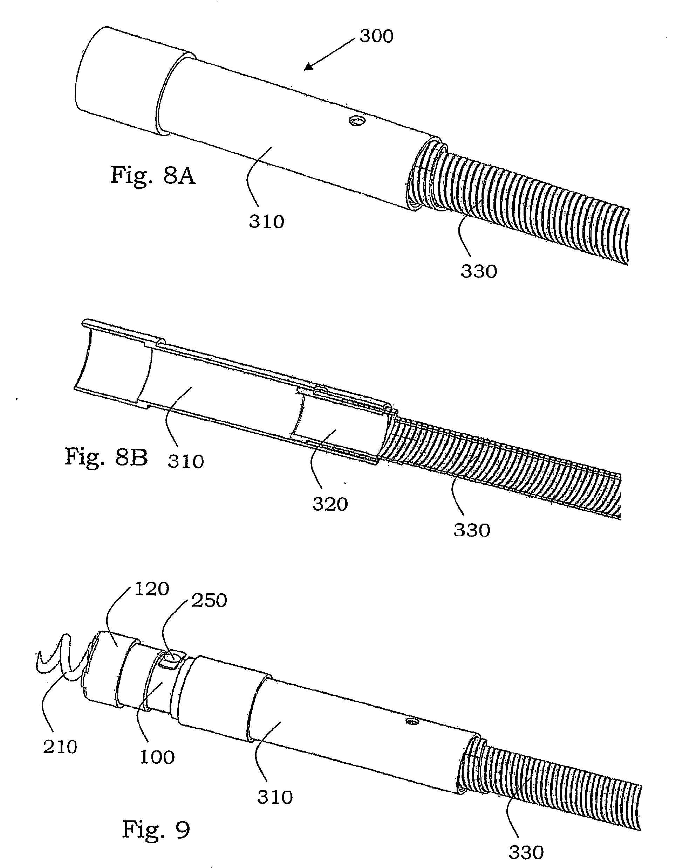

The optional crimp sleeve 320 can be used for interconnecting the ring electrode 310 and the outer conductor 330. In such a case, the portion of the ring electrode 310 being disposed around the crimp sleeve 320 with the conductor 330 therebetween is crimped around the crimp sleeve 330. This crimping mechanically locks the conductor 330 to the ring electrode 310, see Figs. 8A and 8B.

Alternatively, or in addition, the ring electrode 310 is attached to the crimp sleeve 320 through welding or a snap fit locking.

An alternative approach is disclosed in PCT/SE2007/001005, where the crimp sleeve 320 is replaced by a coil adapter. This coil adapter then forms a bridging unit between the ring electrode 310 and the outer coil 330. In such a case, the connection between electrode 310 and adapter and/or the connection between adapter and conductor 330 can be made by spark plasma sintering while the other connection can be made through welding.

A further alternative is to connect the outer conductor 330 directly to the ring electrode 310.

The ring electrode 310 is made of a conducting material, such as platinum, iridium, gold, tantalum, titanium or an alloy such as platinum/iridium or MP35N^{®}.

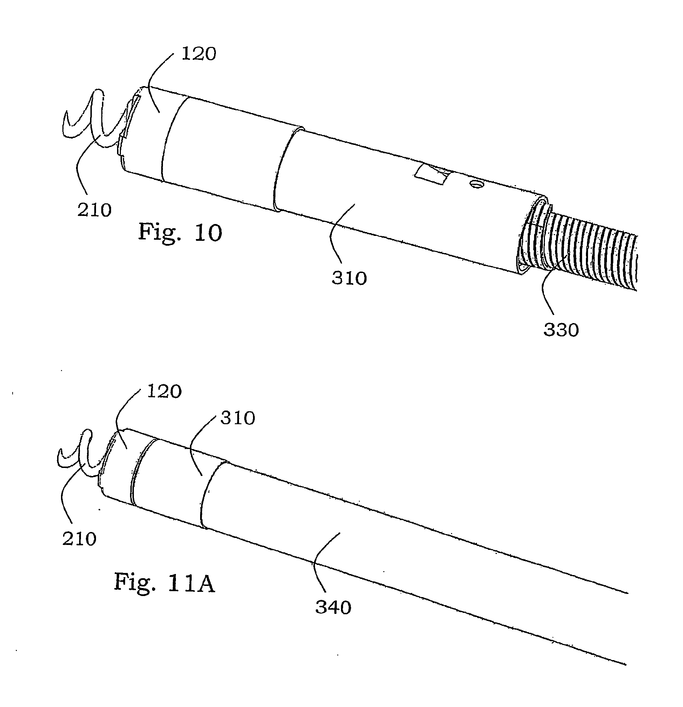

This ring electrode assembly 300 can also be pre-manufactured and handled as a single unit during the lead assembly. The electrode assembly 300 will act as a post locking element by being thread on the inner coil with its insulating tubing and the header assembly 100. Fig. 9 illustrates this process, where the ring electrode assembly is partly thread over header assembly 100 but does not yet cover the post 250. In Fig. 10, the electrode assembly 300 has been fully thread up to the header cap 120 and thereby covers the post and prevents any radial movement of the post.

The post head of the post will further be pressed down towards the lateral surface of the header assembly and preferably into one or more dedicated matching indentations to thereby ensure that the post is fully pushed down against the header assembly.

At this position the ring electrode assembly is locked to the header assembly to prevent any significant longitudinal motion between the two assemblies. Different techniques can be used in this locking, including welding and snap-fit solutions, see PCT/SE2007/000634.

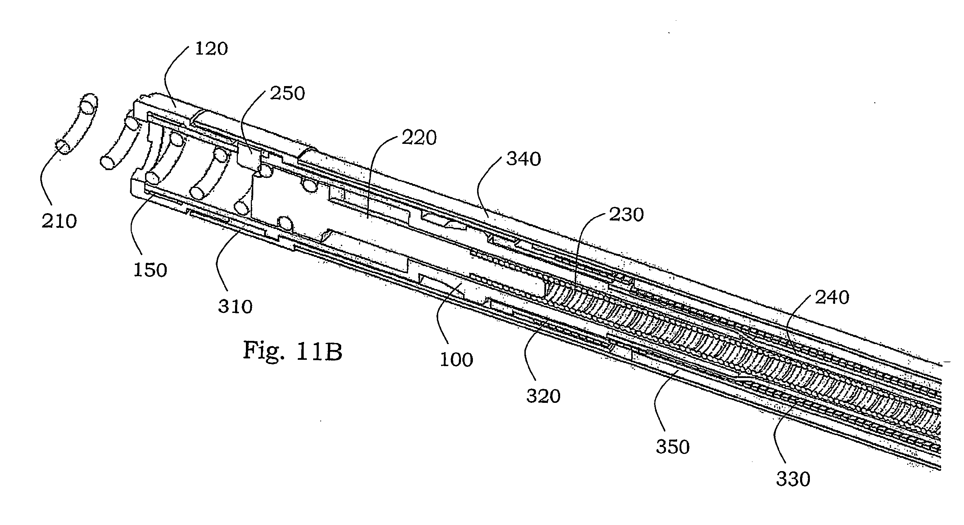

Figs. 11A and 11B illustrate the final lead having an outer insulating tubing 350 thread on outer conductor coil 330. This tubing 350 can be made of a material selected from the group listed as suitable inner tubing materials. Finally, an outer protective sheath 340 can cover the conductors 230, 330 and the insulating tubings 240, 350 and a major portion of the ring electrode except the portion that will face the outside of the lead and function as electrode surface. The sheath 340 can be of different insulating materials, such as OPTIM^{®}, or the like.

The post-locking element that is circumferentially disposed around the portion of the header assembly comprising the radial aperture was the ring electrode assembly in the FSR lead. However, for lead designs in which the ring electrode does not extend so far up to the distal lead end or for leads without any ring electrode, other post-locking elements can be used.

In an embodiment, the radial aperture is positioned close to the most distal end of the header assembly. In such a case, the header cap can be pushed over the post head to thereby prevent any radial movement of the post relative the header assembly.

Another solution is presented in Fig. 12, which is a cross-sectional view of a distal lead portion 2. In this figure, the helix electrode 210 is mechanically and electrically connected to the inner coil conductor 230 by means of the helix shaft 220 manufactured by an electrically conductive material such as platinum, gold, tantalum, titanium or an alloy such as platinum/iridium, e.g. Pt/Ir 90/10 or 80/20. The helix shaft 220 or at least a portion thereof is preferably made of a high density, electrically conductive material to be radiopaque. The lead-connecting (proximal) end of the helix electrode 210 and the distal end of the inner coil conductor 230 may be attached by, for instance laser welding or the like, to the opposite ends of the helix shaft 220. The shaft 220 is journaled for rotation and axial movement within a sleeve 280 and includes a radially extending flange defining a proximal, radially-extending surface engageable against a distal extremity of the sleeve to limit the retraction of the helix electrode 210. The sleeve 280 is preferably electrically conductive and secured to the tubular header assembly 100, which is preferably made of an electrically conductive material in this embodiment. The sleeve 280 and the header assembly 100 are preferably made of an electrically conducting metal, such as titanium, or metal alloy, such as MP35N^{®} or stainless steel.

The proximal portion of the sleeve 280 has a counterbore terminating at a distal end wall. An electrically conductive tubular abutment 260, such as of MP35N^{®} or the like, L-shaped in cross section, has an axial portion connected, e.g. welded, to the proximal end of the helix shaft 220 and a flange projecting radially within the counterbore of the sleeve 280. Thus, the abutment 260 being secured to the shaft 220 is movable rotationally and axially with the shaft 220 relative to the sleeve 280.

Contained within the counterbore is an electrically conductive, expandable/contractable contact member, preferably in the form of a metallic compression spring 270 of, for instance, MP35N^{®} or like material. In such a case, electrically continuity is thereby established between the distal tip (often denoted tip electrode) of header assembly 100 and the terminal contact pin of the connector assembly via the sleeve 250, the contact spring 270, the L-shaped abutment 260 and the inner conductor coil 230. The contact spring 270 is extended or contracted depending on the extension or retraction of the helix electrode 210.

An outer insulating tube 340, for instance of silicone rubber, polyurethane or OPTIM^{®}, extends between the proximal face of the tip electrode and the distal extremity of the ring electrode 310. A corresponding insulating tube 340 also covers the main lead body by extending from the proximal extremity of the ring electrode 310 up to the connector assembly.

The ring electrode 310 is in mechanical and electrical contact with a terminal of the collector assembly, such as the collector ring, through an outer coil conductor 330. The two coil conductors 230, 330 are electrically insulated by a longitudinally extending insulating tube 240, such as made of silicone rubber, polyeurethane or the like. This insulator 240 is disposed between the coils 230, 330 to prevent electrical contact between the conductors 230, 330 and between the ring electrode 310 and the inner conductor coil 230.

An insertable post 250 according to the present invention has been provided in a radial aperture in the header assembly 100 in connection with the distal lead end. The protruding element of the post extends through the radial aperture and projects radially inwardly into the inner header channel and is preferably interposed between adjacent turns of the helical active fixation element 210.

If the lead comprises a marker ring 150, that ring 150 can function as post-locking element to prevent a radial movement of the post 250 out from the aperture. In a particular embodiment, the post 250 can actually be attached to the marker ring 150 as is illustrated in the figure. In such a case, the marker ring 150 constitutes the post head of the post 250 and has a protruding element projecting radially inwardly from an inner surface of the marker ring 150. The post 250 is preferably made of the same material as the marker ring 150 and can therefore be made of a conducting material.

In an alternative embodiment, no post-locking element is required for preventing any radial movement of the post out of the radial aperture. In this embodiment, the post is preferably made of an insulating heat-treatable material, such as a thermoplastic material, including PEEK. In such a case, heat can be applied to the post head after inserting the post into the aperture to fuse the post head with the header assembly material. The header assembly material is then preferably made of the same material as the post to simplify the inter-connection.

Further embodiments use adhesives and/or different welding techniques for locking the post to the header assembly.

In yet another embodiment, at least a portion of the post is threaded to act like a screw. The post is then inserted in the aperture by screwing the post into the aperture. Once the post heads comes into contact with the lateral header surface and the post is fully screwed into the header channel, the screwing action is stopped.

In operation, the post of the present invention acts in the same way as a post fixed to a header as in the art. Thus a rotation of the helical fixation element forces, due to the inserted post, the fixation element to advance or retract within the lead body header.

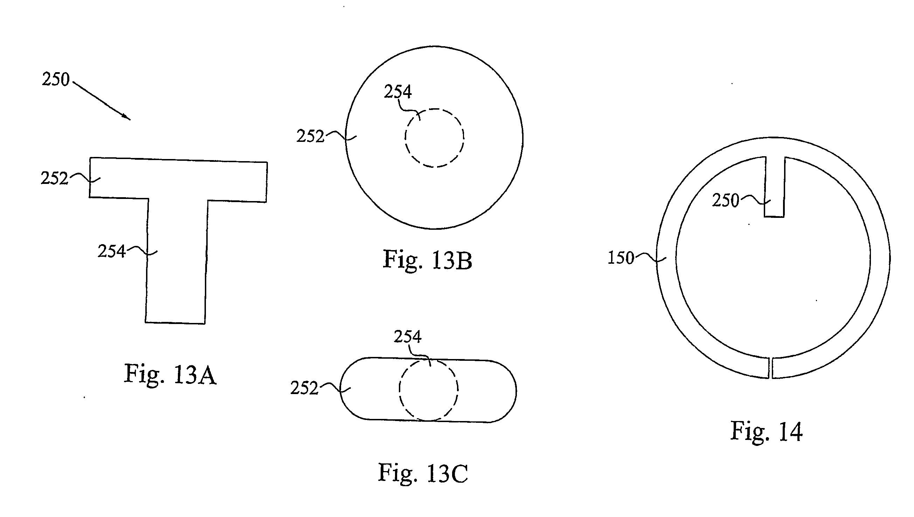

Fig. 13A is a side-view of a post 250 according to an embodiment of the invention. The figure clearly illustrates the preferred design with a post head 252 and connected protruding element 254. As is seen in the figure, the header 252 preferably extends beyond the outer circumferential of the protruding element 254 in at least one dimension to thereby provide a surface that can interact with the lateral surface of the header assembly and prevent the post 250 from falling all the way through the radial aperture.

Figs. 13B and 13C illustrate top-views of different post header 252 embodiments. In Fig. 13B, the header 252 has a general circular shape and can be though of as a disc having a central protruding element 254. In Fig. 13C, the header 252 consists of two wings extending substantially perpendicularly relative the longitudinal axis of the central protruding element 254. This concept can of course be adapted to instead contain only a single wing or utilizing more than two extending wings.

Fig. 14 illustrates the combined post 250 and marker ring 150 solution, in which the marker ring 150 acts as post head with the post 250 protruding radially inwardly towards the centre of the ring 150. In this embodiment, the marker ring 150 preferably does not form a true continuous ring but instead has a discontinuation in the circumference. This allows the marker ring 150 to be disposed around the header assembly by forcing the two non-connecting ends apart and push the marker ring 150 over the assembly and the post 250 in the aperture. The two ends will then snap back toward the original shape to form the ring shape.

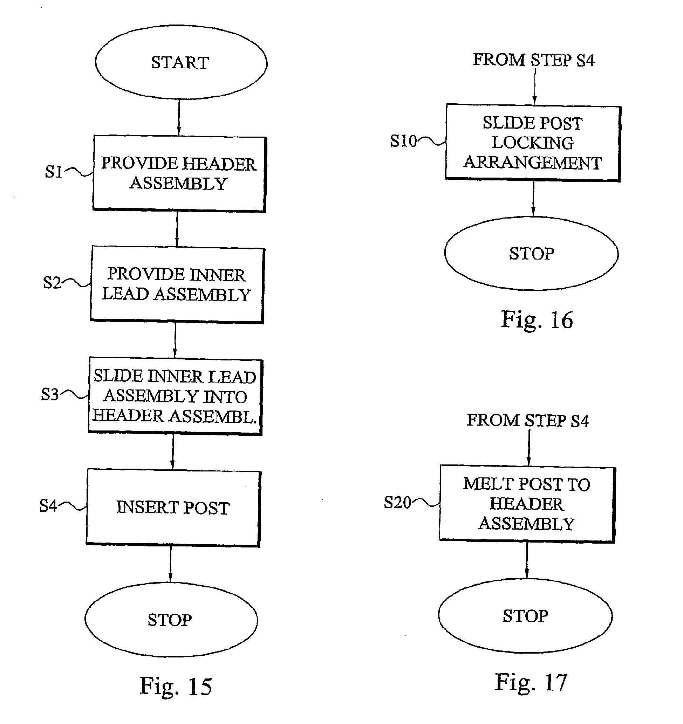

Fig. 15 is a flow diagram of a method of manufacturing an implantable medical lead according to the present invention. The method starts in step S1, where a tubular header assembly is provided. The header assembly (see Fig. 3) defines an inner header channel or bore running from the distal to the proximal ends of the assembly. Furthermore, a radial aperture is provided or can be formed in the header to get a radial channel into the bore. A next step S2 provides an inner lead assembly (see Fig. 4) comprising a helical active fixation element connected to an inner conductor, preferably through a helical shaft. Step S3 slides (see Fig. 5) the inner lead assembly into the inner header channel. This sliding step preferably involves first introducing the proximal end of the inner conductor in the inner header channel from its distal end. The conductor is then fully pushed through the channel until the optional helical shaft and at least a portion of the helical fixation elements enters the channel. The pushing or sliding is preferably stopped once the helical shaft or the helical fixation element comes into contact with the stop in the channel formed by a decrease in the diameter of the header channel going from the distal to the proximal ends of the header assembly. At this position, the post can be inserted into the radial aperture to project radially inwardly into the inner channel. The post prevents the inner lead assembly from leaving the inner channel as the helical shaft cannot be moved longitudinally in the channel past the post.

The post is preferably inserted so that a protruding element of the post becomes interposed between adjacent turns of the helical fixation element. However, if the radial aperture is provided in connection with the most distal end of the lead, the whole helical fixation element might be provided inside the channel in its retracted state so that the post instead protrudes down in connection with the first turn of the helix.

### The method then ends.

It is anticipated by the present invention that the header assembly does not necessarily have to be pre-manufactured with the radial aperture. In such a case, the post is punched or screwed through the header assembly to project into the header channel. This means that the aperture is formed simultaneously as the post penetrates the header. The end of the post that first contacts the lateral surface of the header is preferably pointed or sharp for facilitating the penetration of the header material.

Fig. 16 is a flow diagram illustrating an additional step of the manufacturing method. The method continues from step S4 in Fig. 15. In a next step S 10 a post locking element is slid circumferentially around at least the portion of the tubular header assembly comprising the radial aperture and the post to thereby prevent a radial movement of the post out of the aperture.

Different post locking elements can be used depending on the particular lead design. For instance, a ring electrode can be used in particular with FSR leads. Other lead elements, such as marker ring, header cap, etc. that are provided around the header assembly over the header portion that houses the helical fixation element can alternatively be used.

An insulating adhesive is preferably applied on and preferably partly around the post head. This is in particular useful for FSR leads to thereby prevent any electrical contact between helical fixation element and the ring electrode. Instead of using an insulating adhesive, an insulating, sealing ring can the thread onto the protruding element of the post and pushed up to the post head.

Fig. 17 is a flow diagram illustrating another step of radially locking the post that can be used instead of or as a complement to a post locking element. In this case the post is made of a thermoplastic material that can be caused to partly melt and mechanically inter-connect with the header assembly. Step S20 provides (mild) heat treatment to fuse the post or at least a portion of the post head to the lateral surface of the tubular header assembly.

Another locking solution is to apply and adhesive that glues the post to the header assembly. The type of adhesive utilized is then dependent on the particular material used for the post and the header assembly.

It is also, or instead, possible to lock the post to the header assembly through welding, such as laser or ultrasound welding.

At least a portion of the post can also be threaded so that the post is screwed into the radial aperture. In such a case, no further locking elements or procedures may be required as the screwing is stopped once the post head comes into contact with the outer surface of the header assembly.

It will be understood by a person skilled in the art that various modifications and changes may be made to the present invention without departure from the scope thereof, which is defined by the appended claims.

## Claims

1. An implantable medical lead (1) comprising:
- a tubular header assembly (100) defining an inner header channel (130) running from a first end (110) to a second end (120) of said tubular header assembly (100), said tubular header assembly (100) having a radial aperture (140);
- an inner lead assembly (200) coaxially housed within said inner header channel (130) and comprising a helical active fixation element (210) connected to an inner conductor (230);
- a post (250) in turn comprising:
- a post head (252) mechanically supported by a lateral surface of said tubular header assembly (100); and
- a protruding element (254) connected to said post head (252), extending through said radial aperture (140) and projecting radially inwardly into said inner header channel (130) for transforming a rotation of said helical active fixation element (210) into a longitudinal movement of said helical active fixation element (210) relative said header assembly (100); and
- a post locking element (150; 300) circumferentially disposed around at least a portion of said tubular header assembly (100) comprising said radial aperture (140) to prevent a radial movement of said post (250).

2. The lead according to claim 1, wherein said inner lead assembly (200) comprises said helical active fixation element (210) connected to a first end of a helix shaft (220) and said inner conductor (230) connected to a second end of said helix shaft (220).

3. The lead according to claim 1 or 2, wherein said post (250) projects radially inwardly into said inner header channel (130) and is interposed between adjacent turns of said helical active fixation element (210).

4. The lead according to any of the claims 1 to 3, further comprising an insulating sealing material applied on said post head (252) to form a sealing layer over said post (250) and said radial aperture (140).

5. The lead according to any of the claims 1 to 4, wherein said post locking element (300) is a ring electrode (310) coaxially disposed around said at least a portion of said tubular header assembly (100) and having a proximal end electrically connected to an outer conductor (330) coaxial to said inner conductor (230).

6. The lead according to any of the claims claim 1 to 4, wherein said radial aperture (140) is provided in connection with said first end (110) of said tubular header assembly (100) and said post locking element comprises a header cap (120) circumferentially disposed at said first end (110) of said tubular header assembly (100).

7. The lead according to any of the claims 1 to 4, wherein said radial aperture (140) is provided in connection with said first end (110) of said tubular header assembly (100) and said post locking element (150) comprises a marker ring (150) made of a radiopaque material.

8. The lead according to claim 8, wherein said post (250) is mechanically connected to said marker ring (150) and projects radially inwardly from an inner surface of said marker ring (150).

9. The lead according to any of the claims 1 to 8, wherein said post (250) is made of an insulating material.

10. A method of manufacturing an implantable medical lead (1) comprising the steps of:
- providing a tubular header assembly (100) defining an inner header channel (130) running from a first end (110) to a second end (120) of said tubular header assembly (100), said tubular header assembly (100) having a radial aperture (140);
- providing an inner lead assembly (200) comprising a helical active fixation element (210) connected to an inner conductor (230);
- sliding said inner lead assembly (200) into said inner header channel (130);
- inserting a protruding element (254) of a post (250) connected to a post head (252) into said radial aperture (140) to project radially inwardly into said inner channel (130) for transforming a rotation of said helical active fixation element (210) into a longitudinal movement of said helical active fixation element (210) relative said header assembly (100);
- positioning said post head (252) supported by a lateral surface of said tubular head assembly (100) in connection with said radial aperture (140); and
- sliding a post locking element (150; 300) circumferentially around at least a portion of said tubular header assembly (100) comprising said radial aperture (140) to prevent a radial movement of said post (250).

11. The method according to claim 10, wherein said inserting step comprises inserting said post (250) into said radial aperture (140) to project radially inwardly into said inner channel (130) and interpose between adjacent turns of said helical active fixation element (210).

12. The method according to claim 10 or 11, further comprising applying an insulating adhesive on said post head (252) to form a sealing layer over said post (250) and said radial aperture (140).

## Patentansprüche

1. Implantierbare medizinische Leitung (1), enthaltend:
eine röhrenförmige Kopfanordnung (100), die einen inneren Kopfkanal (130) definiert, der von einem ersten Ende (110) zu einem zweiten Ende (120) der röhrenförmigen Kopfanordnung (100) verläuft, wobei die röhrenförmige Kopfanordnung (100) eine radiale Öffnung (140) aufweist;
eine innere Leitungsanordnung (200), die koaxial innerhalb des inneren Kopfkanals (130) untergebracht ist und ein spiralförmiges aktives Befestigungsbauteil (210) enthält, das mit einem inneren Leiter (230) verbunden ist;
einen Stift (250), der enthält:
einen Stiftkopf (252), der durch eine laterale Fläche der röhrenförmigen Kopfanordnung (100) mechanisch abgestützt ist; und
ein vorstehendes Bauteil (254), das mit dem Stiftkopf (252) verbunden ist, sich durch die radiale Öffnung (140) erstreckt und radial nach innen in den inneren Kopfkanal (130) vorsteht zur Transformation einer Drehung des spiralförmigen aktiven Befestigungsbauteils (210) in eine Längsbewegung des spiralförmigen aktiven Befestigungsbauteils (210) relativ zu der Kopfanordnung (100); und
ein Stiftverriegelungsbauteil(150; 300), das umfangsmäßig um mindestens einen Bereich der röhrenförmigen Kopfanordnung (100) herum angeordnet ist, die die radiale Öffnung (140) enthält, zum Verhindern einer radialen Bewegung des Stifts (250).

2. Leitung nach Anspruch 1, bei der die innere Leitungsanordnung (200) das spiralförmige aktive Befestigungsbauteil (210), das mit einem ersten Ende einer spiralförmigen Welle (220) verbunden ist, und eine innere Leitung (230), die mit einem zweiten Ende der spiralförmigen Welle (220) verbunden ist, enthält.

3. Leitung nach Anspruch 1 oder 2, bei der der Stift (250) radial nach innen in den inneren Kopfkanal (130) vorsteht und zwischen benachbarten Windungen des spiralförmigen aktiven Befestigungsbauteils (210) angeordnet ist.

4. Leitung nach einem der Ansprüche 1 bis 3, ferner enthaltend ein isolierendes Dichtungsmaterial, das auf dem Stiftkopf (250) aufgebracht ist zum Bilden einer Dichtungsschicht über dem Stift (250) und der radialen Öffnung (140).

5. Leitung nach einem der Ansprüche 1 bis 4, bei der das Stiftverriegelungsbauteil (300) eine Ringelektrode (310) ist, die koaxial um den mindestens einen Bereich der röhrenförmigen Kopfanordnung (100) herum angeordnet ist und ein proximales Ende aufweist, das mit einer äußeren Leitung (330), die koaxial zu der inneren Leitung (230) ist, elektrisch verbunden ist.

6. Leitung nach einem der Ansprüche 1 bis 4, bei der die radiale Öffnung (140) in Verbindung mit dem ersten Ende (110) der röhrenförmigen Kopfanordnung (100) bereitgestellt ist, und das Stiftverriegelungsbauteil eine Kopfkappe (120) enthält, die umfangsmäßig an dem ersten Ende (110) der röhrenförmigen Kopfanordnung (100) angeordnet ist.

7. Leitung nach einem der Ansprüche 1 bis 4, bei der die radiale Öffnung (140) in Verbindung mit dem erste Ende (110) der röhrenförmigen Kopfanordnung (100) bereitgestellt ist und das Stiftverriegelungsbauteil (150) einen Markierungsring (150) enthält, der aus einem radiopaquen Material gebildet ist.

8. Leitung nach Anspruch 7, wobei der Stift (250) mechanisch mit dem Markierungsring (150) verbunden ist und radial nach innen von einer inneren Fläche des Markierungsrings (150) weg steht.

9. Leitung nach einem der Ansprüche 1 bis 8, bei der der Stift (250) aus einem Isolationsmaterial ist.

10. Verfahren zum Herstellen einer implantierbaren medizinischen Leitung, enthaltend die Schritte:
Bereitstellen einer röhrenförmigen Kopfanordnung (100), die einen inneren Kopfkanal (130) definiert, der von einem ersten Ende (110) zu einem zweiten Ende (120) der röhrenförmigen Kopfanordnung (100) verläuft, wobei die röhrenförmige Kopfanordnung (100) eine radiale Öffnung (140) enthält;
Bereitstellen einer inneren Leitungsanordnung (200), die ein spiralförmiges aktives Befestigungsbauteil (210) enthält, das mit einem inneren Leiter (230) verbunden ist;
Schieben der inneren Leitungsanordnung (200) in den inneren Kopfkanal (130);
Einführen eines vorstehenden Bauteils (254) eines Stifts (250), der mit einem Stiftkopf (252) verbunden ist, in die radiale Öffnung (140), um radial nach innen in den inneren Kanal (130) vorzustehen, zum Transformieren einer Drehung des spiralförmigen aktiven Befestigungsbauteils (210) in einer Längsbewegung des spiralförmigen aktiven Befestigungsbauteils (210) relativ zu der Kopfanordnung (100);
Positionieren des Stiftkopfs (252), der durch eine laterale Fläche der röhrenförmigen Kopfanordnung (100) in Verbindung mit der radialen Öffnung (140) abgestützt wird; und
Schieben eines Stiftverriegelungsbauteils (150; 300) umfangsmäßig um mindestens einen Bereich der röhrenförmigen Kopfanordnung (100) herum, die die radiale Öffnung (140) enthält, um eine radiale Bewegung des Stifts (250) zu verhindern.

11. Verfahren nach Anspruch 10, bei dem der Einführungsschritt ein Einführen des Stifts (250) in die radiale Öffnung (140) enthält, um radial nach innen in den inneren Kanal (130) vorzustehen und zwischen benachbarten Windungen des spiralförmigen aktiven Befestigungsbauteils (210) angeordnet ist.

12. Verfahren nach Anspruch 10 oder 11, ferner mit einem Aufbringen eines isolierenden Klebers auf den Stiftkopf (252), um eine Abdichtungsschicht über dem Stift (250) und der radialen Öffnung (140) zu bilden.

## Revendications

1. Dérivation (1) médicale implantable, comprenant :
- un ensemble (100) tubulaire d'en-tête définissant un canal (130) intérieur d'en-tête d'une première extrémité (110) à une deuxième extrémité (120) de l'ensemble (100) tubulaire d'en-tête, l'ensemble (100) tubulaire d'en-tête ayant une ouverture (140) radiale ;
- un ensemble (200) intérieur de dérivation logé coaxialement dans le canal (130) intérieur d'en-tête et comprenant un élément (210) hélicoïdal actif de fixation relié à un conducteur (230) intérieur ;
- un pilier (250) comprenant à son tour :
- une tête (252) de pilier supportée mécaniquement par une surface latérale de l'ensemble (100) tubulaire d'entête ; et
- un élément (254) en saillie relié à la tête (252) de pilier, passant dans l'ouverture (140) radiale et faisant saillie radialement vers l'intérieur dans le canal (130) intérieur d'en-tête, pour transformer une rotation de l'élément (210) hélicoïdal actif de fixation en un mouvement longitudinal de l'élément (210) hélicoïdal actif de fixation par rapport à l'ensemble (100) d'en-tête ; et
- un élément (150, 300) de verrouillage du pilier disposé circonférentiellement autour d'au moins une partie de l'ensemble (100) tubulaire d'en-tête, comprenant l'ouverture (140) radiale pour empêcher un mouvement radial du pilier (250) .

2. Dérivation suivant la revendication 1, dans laquelle l'ensemble (200) intérieur de dérivation comprend l'élément (210) hélicoïdal actif de fixation relié à une première extrémité d'un arbre (220) d'hélice et le conducteur (230) intérieur relié à une deuxième extrémité de l'arbre (200) d'hélice.

3. Dérivation suivant la revendication 1 ou 2, dans laquelle le pilier (250) fait saillie radialement vers l'intérieur dans le canal (130) intérieur d'en-tête et est interposé entre des spires voisines de l'élément (210) hélicoïdal actif de fixation.

4. Dérivation suivant l'une quelconque des revendications 1 à 3, comprenant, en outre, une matière isolante de scellement appliquée sur la tête (252) du pilier pour former une couche de scellement sur le pilier (250) et sur l'ouverture (140) radiale.

5. Dérivation suivant l'une quelconque des revendications 1 à 4, dans laquelle l'élément (300) de verrouillage du pilier est une électrode (310) annulaire disposée coaxialement autour d'au moins une partie de l'ensemble (100) tubulaire d'en-tête et ayant une extrémité proximale reliée électriquement à un conducteur (330) extérieur coaxial au conducteur (230) intérieur.

6. Dérivation suivant l'une quelconque des revendications 1 à 4, dans laquelle l'ouverture (140) axiale est prévue en liaison avec la première extrémité (110) de l'ensemble (100) tubulaire d'en-tête et l'élément de verrouillage du pilier comprend un capuchon (120) d'en-tête disposé circonférentiellement à la première extrémité (110) de l'ensemble (100) tubulaire d'entête.

7. Dérivation suivant l'une quelconque des revendications 1 à 4, dans laquelle l'ouverture (140) radiale est prévue en liaison avec la première extrémité (110) de l'ensemble (100) tubulaire d'en-tête et l'élément (150) de verrouillage de pilier comprend un anneau (150) marqueur en un matériau radio-opaque.

8. Dérivation suivant la revendication 7, dans laquelle le pilier (250) est relié mécaniquement à l'anneau (150) marqueur et fait saillie radialement vers l'intérieur d'une surface intérieure de l'anneau (250) marqueur.

9. Dérivation suivant l'une quelconque des revendications 1 à 8, dans laquelle le pilier (250) est en un matériau isolant.

10. Procédé de fabrication d'une dérivation (1) médicale implantable comprenant les stades, dans lesquels :
- on se procure un ensemble (100) tubulaire d'en-tête définissant un canal (130) intérieur d'en-tête allant d'une première extrémité (110) à une deuxième extrémité (120) de l'ensemble (100) tubulaire d'en-tête, l'ensemble (100) tubulaire d'en-tête ayant une ouverture (140) radiale ;
- on se procure un ensemble (200) intérieur de dérivation comprenant un élément (210) hélicoïdal actif de fixation relié à un conducteur (230) intérieur ;
- on fait coulisser l'ensemble (200) intérieur de dérivation dans le canal (130) intérieur d'en-tête ;
- on insère un élément (250) en saillie d'un pilier (250) relié à une tête (252) de pilier dans l'ouverture (140) radiale pour qu'il fasse saillie radialement vers l'intérieur dans le canal (130) intérieur afin de transformer une rotation de l'élément (210) hélicoïdal actif de fixation en un mouvement longitudinal de l'élément (210) hélicoïdal actif de fixation par rapport à l'ensemble (100) d'en-tête ;
- on met en position la tête (250) de pilier supportée par une surface latérale de l'ensemble (100) tubulaire d'entête en liaison avec l'ouverture (140) radiale ; et
- on fait coulisser un élément (150, 300) de verrouillage de pilier circonférentiellement autour d'au moins une partie de l'ensemble (100) tubulaire d'en-tête, comprenant l'ouverture (140) radiale pour empêcher un mouvement radial du pilier (250).

11. Procédé suivant la revendication 10, dans lequel le stade d'insertion comprend l'insertion du pilier (250) dans l'ouverture (140) radiale pour qu'il fasse saillie radialement vers l'intérieur dans le canal (130) intérieur et s'interpose entre des spires voisines de l'élément (210) hélicoïdal actif de fixation.

12. Procédé suivant la revendication 10 ou 11, comprenant, en outre, l'application d'un adhésif isolant sur la tête (252) du pilier pour former une couche de scellement sur le pilier (250) et sur l'ouverture (140) radiale.
